(19) Europäisches Patentamt
European Patent Office
Office européen des brevets

(11) **EP 1 314 028 B1**

(12) **EUROPÄISCHE PATENTSCHRIFT**

(45) Veröffentlichungstag und Bekanntmachung des Hinweises auf die Patenterteilung:
**21.02.2007 Patentblatt 2007/08**

(21) Anmeldenummer: **01964905.2**

(22) Anmeldetag: **16.08.2001**

(51) Int Cl.:
***G01N 33/20*** *(2006.01)*

(86) Internationale Anmeldenummer:
**PCT/DE2001/003127**

(87) Internationale Veröffentlichungsnummer:
**WO 2002/018934 (07.03.2002 Gazette 2002/10)**

(54) **VERFAHREN ZUR BESTIMMUNG DER THERMISCHEN MATERIALEIGENSCHAFTEN VON METALL-FORMTEILEN**

METHOD FOR DETERMINING THE THERMAL MATERIAL PROPERTIES OF METAL SHAPED PARTS

PROCEDE POUR DETERMINER LES PROPRIETES THERMIQUES DU MATERIAU CONSTITUANT DES PIECES MOULEES EN METAL

(84) Benannte Vertragsstaaten:
**AT BE CH CY DE DK ES FI FR GB GR IE IT LI LU MC NL PT SE TR**

(30) Priorität: **29.08.2000 DE 10042386**

(43) Veröffentlichungstag der Anmeldung:
**28.05.2003 Patentblatt 2003/22**

(73) Patentinhaber: **SIEMENS AKTIENGESELLSCHAFT**
**80333 München (DE)**

(72) Erfinder:
• **GRAMCKOW, Otto**
  **91080 Uttenreuth (DE)**
• **JANSEN, Michael**
  **91096 Möhrendorf (DE)**
• **POST, Martin**
  **47608 Geldern (DE)**
• **WEINZIERL, Klaus**
  **91052 Erlangen (DE)**

(56) Entgegenhaltungen:
JP-A- 4 080 324       JP-A- 4 232 214
US-A- 5 893 055       US-A- 6 044 895

**Beschreibung**

Die Erfindung betrifft ein Verfahren zur Bestimmung der thermischen Materialeigenschaften von Metall-Formteilen.

Bei bestimmten technologischen Prozessen, wie z. B. der Abkühlung von warmgewalzten Stahlbändern, ist eine möglichst genaue Bestimmung der thermischen Materialeigenschaften der Metall-Formteile notwendig bzw. wünschenswert. Bei der Abkühlung von warmgewalzten Stahlbändern werden während des Durchlaufens der Kühlstrecke der Warmwalzstraße die Gefügeeigenschaften der gewalzten Stahlbänder eingestellt, wobei die Einstellung der Gefügeeigenschaften primär durch die zugeführte Wassermenge erfolgt. Zur Berechnung der erforderlichen Wassermenge wird ein Kühlstreckenmodell auf der Basis der Fourier'schen Wärmeleitungsgleichung eingesetzt. Mit diesem Modell kann die Temperaturverteilung im Stahlband während des Abkühlvorganges berechnet werden.

Eine Schwierigkeit bei der Modellierung stellt die relativ starke Abhängigkeit der thermischen Materialeigenschaften von den zugesetzten Legierungselementen dar. Beispielsweise sinkt die Wärmeleitfähigkeit bei 500° C durch Zusetzen von 1 % Chrom auf etwa den halben Wert ab.

Aufgrund der relativ starken Abhängigkeit der thermischen Materialeigenschaften von den zugesetzten Legierungselementen müssen letztere relativ genau bestimmt werden. Dies geschieht bisher durch Messungen im thermodynamischen Gleichgewicht. Der konkrete Ansatz für die Chemieabhängigkeit kann beispielsweise dadurch erfolgen, dass man die Wärmeleitfähigkeit des Materials, die eine Funktion der Temperatur ist, mittels eines Polygonzuges beschreibt. Die Koordinaten der dazugehörigen Stützstellen werden hierbei von den im Material enthaltenen Legierungselementen abhängig gemacht. Diese auf der jeweiligen Anlage direkt bestimmten Abhängigkeiten sind tabellarisch erfasst. Im einfachsten Fall arbeitet man hierbei mit der Summe der Legierungselemente. Freie Parameter werden durch eine Ausgleichsrechnung anhand von Messungen ermittelt.

Bei dem beschriebenen Verfahren ausreichend genaue Werte zu erhalten ist sehr zeitaufwendig.

Aufgabe der Erfindung ist es, ein Verfahren zu schaffen, dass es mit einem geringen Zeitaufwand ermöglicht, eine ausreichend genaue Bestimmung der thermischen Materialeigenschaften zu ermöglichen.

Die Aufgabe wird erfindungsgemäß durch die Merkmale des Anspruchs 1 gelöst. Vorteilhafte Ausgestaltungen der Erfindung sind jeweils in den weiteren Ansprüchen beschrieben.

Ähnliche Verfahren sind den Druckschriften US-A-6 044 895 und JP-A-4 232 214 zu entnehmen.

Bei dem erfindungsgemäßen Verfahren werden die thermischen Materialeigenschaften von Metall-Formteilen aus einem Modell bestimmt, das die thermischen Materialeigenschaften des Metall-Formteils beschreibt, wobei

- wenigstens ein thermodynamischer Parameter p als Linearkombination aus wenigstens einer Basisfunktion $h_i$ und wenigstens einem Gewichtungsfaktor $g_i$ gemäß der Beziehung

$$p = \pm \sum_{i=1}^{n} g_i \cdot h_i + c, \quad n \in N, \, c = \text{const.}$$

oder

$$p = \pm \sum_{i=1}^{n} (g_i + c_i) \cdot h_i, \quad n \in N, \, c_i = \text{const.}$$

gebildet wird und

- die Basisfunktion $h_i$ die thermischen Materialeigenschaften beschreibt und
- der Gewichtungsfaktor $g_i$ den Einfluss der Legierungselemente auf wenigstens einen thermodynamischen Parameter p berücksichtigt.

Das Verfahren nach Anspruch 1 stellt eine Kombination eines physikalischen Modells und datengestützter Methoden zur Darstellung eines Materialmodells dar, wobei die thermischen Materialeigenschaften erfindungsgemäß durch wenigstens eine Linearkombination aus wenigstens einer Basisfunktion $h_i$ und wenigstens einem Gewichtungsfaktor $g_i$ dargestellt werden. Damit ist bei dem erfindungsgemäßen Verfahren kein konkreter Ansatz für die schwer zu beschreibende Abhängigkeit von den Legierungselementen erforderlich. Darüber hinaus kann die Berücksichtigung der Legierungselemente sehr differenziert erfolgen. Dadurch kann bei ausreichender Anzahl von Messdaten ein sehr genaues Materialmodell gewonnen werden.

Die thermischen Materialeigenschaften, die durch den thermodynamischen Parameter p definiert sind und die durch wenigstens eine Basisfunktion beschrieben werden können, sind beispielsweise die Enthalpie, die Wärmekapazität und die Wärmeleitfähigkeit.

Im Rahmen der Erfindung können als Basisfunktionen, alternativ oder in Kombination, beispielsweise Rechteckblöcke, Sägezähne, B-Splines, vorzugsweise B-Splines 4. Ordnung, oder Gaußglocken verwendet werden (Ansprüche 3 bis 6).

Besteht die Linearkombination ausschließlich aus Rechteckblöcken (Anspruch 3), dann ergibt sich für den thermodynamischen Parameter p eine Stufenfunktion.

Bei einer ausschließlichen Linearkombination von Sägezähnen (B-Splines 1. Ordnung, Anspruch 4) erhält man für den thermodynamischen Parameter p einen Polygonzug.

Werden ausschließlich B-Splines 4. Ordnung zur Bildung einer Linearkombination benutzt (Anspruch 5), dann erhält man für den thermodynamischen Parameter p einen kubischen Spline.

Bei einer vorteilhaften Ausgestaltung des erfindungsgemäßen Verfahrens werden die Gewichtungsfaktoren $g_i$ in einem neuronalen Netz aus den Massenanteilen der zugesetzten Legierungselemente und/oder daraus abgeleiteter Größen bestimmt (Anspruch 7).

Zum Training des neuronalen Netzes wird eine vorgebbare Zahl von Datensätzen herangezogen, die jeweils für ein bestimmtes warmgewalztes Stahlband Messungen von Oberflächentemperaturen und Geschwindigkeit sowie Angaben über die zur Kühlung benötigten Wassermengen beinhalten. Die jeweiligen Stahlbänder können hierbei - müssen aber nicht - unterschiedliche Konzentrationen von Legierungselementen aufweisen.

Die Datensätze beinhalten Messungen für zumindest näherungsweise Reineisen. Aus diesen Teilen der Datensätze wird ein Modell ermittelt und an die Realität angepasst. Die thermodynamischen Parameter für Reineisen sind bekannt, weshalb eine Anpassung des Modells an die Realität nur noch für den Wärmeübergang durchgeführt wird. Anschließend wird der gesamte Datensatz betrachtet, der auch Messungen für legierte Stähle beinhaltet. Aus diesem Datensatz wird eine mögliche Abweichung von dem zuvor an die Realität angepassten Modell bestimmt. Diese Abweichung ist nur aufgrund von im Material vorhandenen Legierungsanteilen - und nicht aufgrund des Abkühlvorgangs (Wassermenge und Wassertemperatur) zu erklären. Die Anpassung der Berechnungen an die Messungen erfolgt durch die Veränderung der Netzgewichte $w_i$. Die Veränderung der Netzgewichte $w_i$ führt zu einer Änderung der Gewichtungsfaktoren $g_i$. Die Anpassung der Berechnungen an die Messungen wird so lange vorgenommen, bis für alle Formteile (z.B. Stahlbänder) die Berechnungen hinreichend gut mit den Messungen übereinstimmen.

Alternativ zu einer Bestimmung von Gewichtungsfaktoren $g_i$ in einem neuronalen Netz können Gewichtungsfaktoren $g_i$ durch eine Linearkombination aus dem Massenanteil wenigstens eines Legierungselementes und/oder daraus abgeleiteter Größen sowie einem Regressionsfaktor ermittelt werden (Anspruch 8).

Weitere vorteilhafte Ausgestaltungen der Erfindung werden nachfolgend anhand von zwei in der Zeichnung dargestellten Ausführungsbeispielen näher erläutert. Es zeigen in Prinzipdarstellung:

FIG 1 ein Blockdiagramm einer ersten Ausführungsform des erfindungsgemäßen Verfahrens,
FIG 2 eine gegenüber dem Verfahren nach FIG 1 alternative Ermittlung der Gewichtungsfaktoren,
FIG 3 eine gegenüber dem Verfahren nach FIG 1 und FIG 2 alternative Ermittlung der Gewichtungsfaktoren.

Bei dem erfindungsgemäßen Verfahrens werden die thermischen Materialeigenschaften von Metall-Formteilen aus einem Modell bestimmt, das die thermischen Materialeigenschaften des Metall-Formteils beschreibt, wobei

- wenigstens ein thermodynamischer Parameter p als Linearkombination aus wenigstens einer Basisfunktion $h_i$ und wenigstens einem Gewichtungsfaktor $g_i$ gemäß der Beziehung

$$p = \pm \sum_{i=1}^{n} g_i \cdot h_i + c, \quad n \in N, c = \text{const.}$$

oder

$$p = \pm \sum_{i=1}^{n} (g_i + c_i) \cdot h_i, \quad n \in N, \, c_i = \text{const.}$$

gebildet wird und
- die Basisfunktion $h_i$ die thermischen Materialeigenschaften beschreibt und
- der Gewichtungsfaktor $g_i$ den Einfluss der Legierungselemente auf wenigstens einen thermodynamischen Parameter p berücksichtigt.

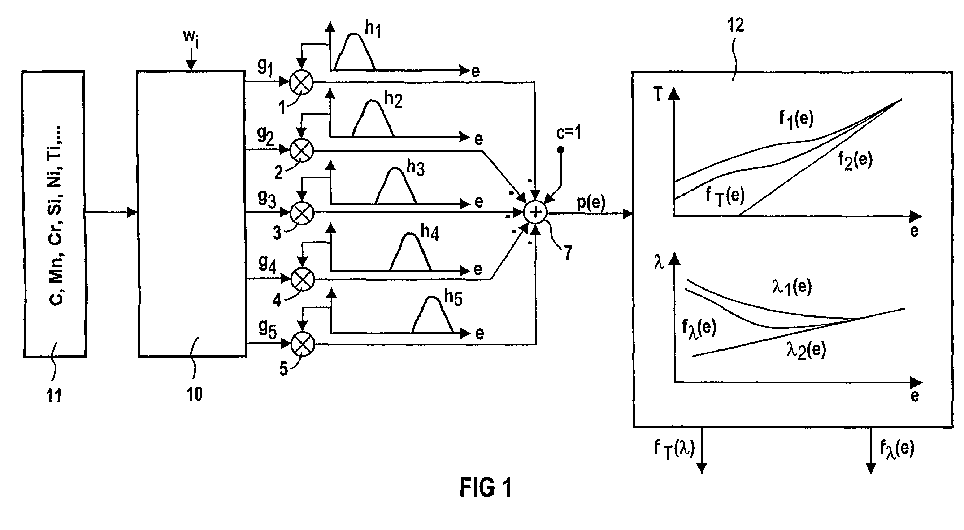

Bei dem in FIG 1 gezeigten Ausführungsbeispiel werden die thermischen Materialeigenschaften von warmgewalzten Stahlbändern bestimmt. Alle fünf Basisfunktionen $h_1$ bis $h_5$ sind bei der dargestellten Ausführungsform B-Splines 4. Ordnung. Für den thermodynamischen Parameter p, der die thermischen Materialeigenschaften des Formteils beschreibt, erhält man damit einen Spline bestehend aus Polynomen 3. Grades.

Bei der durch den thermodynamischen Parameter p beschriebenen Materialeigenschaft handelt es sich bei dem in FIG 1 dargestellten Ausführungsbeispiel um den Umwandlungsgrad von Austenit nach Ferrit in Abhängigkeit von der Enthalpie e. Im Rahmen der Erfindung ist es jedoch beispielsweise auch möglich, thermische Materialeigenschaften durch die Wärmekapazität und/oder die Wärmeleitfähigkeit zu beschreiben.

Bei der Ausgestaltung gemäß FIG 1 werden die Gewichtungsfaktoren $g_1$ bis $g_5$ in einem neuronalen Netz 10 aus den Massenanteilen der dem Stahl zugesetzten Legierungselemente (C, Mn, Cr, Si, Ni, Ti, ...) und den Netzgewichten $w_i$ bestimmt. Die Netzgewichte $w_i$ werden durch Training des neuronalen Netzes 10 (auch als Optimierung bezeichnet) gewonnen und/oder von einem in FIG 1 nicht gezeigten Datenspeicher dem neuronalen Netz 10 zugeführt.

Die dem Stahl zugesetzten Legierungselemente werden als Datensätze von einem Datenspeicher 11 zur Verfügung gestellt. Zum Training des neuronalen Netzes 10 wird eine vorgebbare Zahl dieser Datensätze herangezogen, die neben den Legierungsanteilen für ein bestimmtes warmgewalztes Stahlband auch die Messungen von Oberflächentemperaturen und Angaben über die zur Kühlung benötigten Wassermengen beinhalten. Die jeweiligen Stahlbänder können hierbei auch unterschiedliche Konzentrationen von Legierungselementen aufweisen.

Jede der als B-Splines 4. Ordnung zur Verfügung stehenden Basisfunktionen $h_1$ bis $h_5$ wird in einem Multiplizierer 1 bis 5 mit jeweils einem Gewichtungsfaktor $g_1$ bis $g_5$ sowie mit (-1) multipliziert und einem Summierer 6 zugeführt. Dem Summierer 6 wird im dargestellten Ausführungsbeispiel außerdem noch für die legierungsunabhängige Konstante c der Wert c = 1 zugeführt. Am Ausgang des Summierers 6 steht damit für p(e) der Wert

$$p(e) = - \sum_{i=1}^{5} g_i \cdot h_i + 1,$$

an.

Mit p(e) als Umwandlungsgrad von Austenit nach Ferrit (bzw. Perlit) in Abhängigkeit von der Enthalpie e ergibt sich der folgende, im Funktionsblock 12 ermittelten funktionale Zusammenhang zwischen der Enthalpie e und der Temperatur T:

$$f_T(e) = p(e) \cdot f_1(e) + [1 - p(e)] \cdot f_2(e).$$

Für die Wärmeleitfähigkeit $\lambda$ und die Enthalpie e erhält man dann den folgenden, ebenfalls im Funktionsblock 12 ermittelten funktionalen Zusammenhang:

$$f_\lambda(e) = p(e) \cdot \lambda_1(e) + [1 - p(e)] \cdot \lambda_2(e).$$

Sind alle Gewichtungsfaktoren $g_i = 0$, sind also dem Stahl keine Legierungselemente beigemischt, dann wird $p(e) \equiv 1$, es liegt also reiner Ferrit bzw. Perlit vor.

Für den funktionalen Zusammenhang zwischen der Enthalpie e und der Temperatur T erhält man damit die Beziehung

$$f_T(e) = f_1(e).$$

Für den funktionalen Zusammenhang zwischen der Wärmeleitfähigkeit $\lambda$ und der Enthalpie e ergibt sich dann die Beziehung

$$f_\lambda(e) = \lambda_1(e).$$

Demgegenüber erhält man für $p(e) \equiv 0$, d.h. für reinen Austenit, die folgenden funktionalen Zusammenhänge:

$$f_T(e) = f_2(e).$$

$$f_\lambda(e) = \lambda_2(e).$$

Definiert man p(e) jedoch als Restaustenitgehalt, und nicht (wie im Ausführungsbeispiel gemäß FIG 1) als Umwandlungsgrad von Austenit nach Ferrit, dann ist für die Beziehung

$$p = \sum_{i=1}^{n} g_i \cdot h_i + c, \quad n \in N,$$

mit c = 0 zu wählen.

Für $p(e) \equiv 0$ liegt dann reiner Ferrit bzw. Perlit vor, wohingegen für $p(e) \equiv 1$ reiner Austenit vorliegt.

Bei den funktionalen Zusammenhängen für $f_T(e)$ und $f_\lambda(e)$ sind dann die Indizes 1 und 2 zu vertauschen.

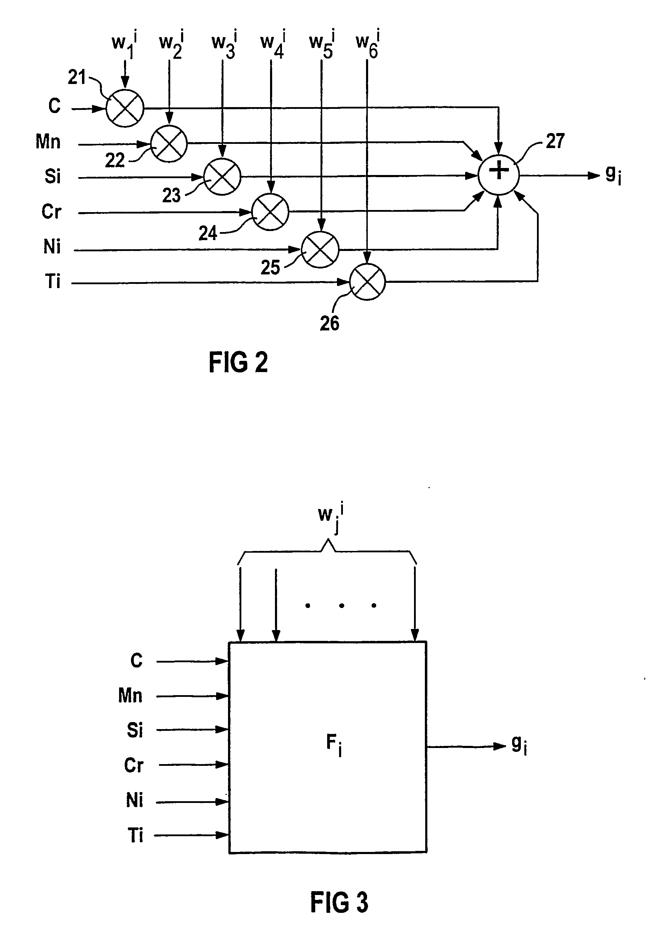

Alternativ zu einer Bestimmung eines Gewichtungsfaktors $g_i$ in einem neuronalen Netz 10 kann dieser Gewichtungsfaktor $g_i$ auch durch eine Linearkombination aus den Massenanteilen der Legierungselemente C, Mn, Cr, Si, Ni, Ti mit jeweils einem Regressionsfaktor $w_1^i$ bis $w_6^i$ ermittelt werden. Gemäß FIG 2 werden die sechs Linearkombinationen in jeweils einem Multiplizierer 21 bis 26 ermittelt und einem Summierer 27 zugeführt, der daraus einen Gewichtungsfaktor $g_i$ ermittelt.

Dieser Gewichtungsfaktor $g_i$ wird auf die in FIG 1 gezeigte Weise im Funktionsblock 12 weiterverarbeitet.

Alternativ zu einer Bestimmung eines Gewichtungsfaktors $g_i$ in einem neuronalen Netz 10 (FIG 1) oder einer Linearkombination (FIG 2) kann dieser Gewichtungsfaktor $g_i$ auch aus den Massenanteilen der Legierungselemente C, Mn, Cr, Si, Ni, Ti mit mindestens einem freien Parameter $w_j^i$ mittels einer nichtlinearen Funktion gebildet werden.

Gemäß FIG 3 werden die sechs Legierungselemente sowie die freien Parameter $w_j^i$ der nichtlinearen Funktion $F_i$ mehrerer Veränderlicher zugeführt. Das Ergebnis der nichtlinearen Funktion $F_i$ ist der Gewichtungsfaktor $g_i$.

Alternativ können auch Legierungselemente hinzugefügt und/oder entfernt werden. Beispielsweise kann auch eine Kombination C, Mn, Cr, Si, V verwendet werden.

**Patentansprüche**

1. Verfahren zur Bestimmung der thermischen Materialeigenschaften von Metall-Formteilen aus einem Modell, das die thermischen Materialeigenschaften des Metall-Formteils beschreibt, wobei

   - wenigstens ein thermodynamischer Parameter (p) als Linearkombination aus wenigstens einer Basisfunktion ($h_i$) und wenigstens einem Gewichtungsfaktor ($g_i$) gemäß der Beziehung

$$p = \pm \sum_{i=1}^{n} g_i \cdot h_i + c, \quad n \in N, c = \text{const.}$$

   oder

$$p = \pm \sum_{i=1}^{n} (g_i + c_i) \cdot h_i, \quad n \in N, c_i = \text{const.}$$

   gebildet wird und
   - die Basisfunktion ($h_i$) die thermischen Materialeigenschaften beschreibt und
   - der Gewichtungsfaktor ($g_i$) den Einfluss der Legierungselemente auf wenigstens einen thermodynamischen Parameter (p) berücksichtigt.

2. Verfahren nach Anspruch 1, wobei

   - es sich bei den thermischen Materialeigenschaften, welche durch wenigstens eine Basisfunktion beschrieben werden, um die Temperatur und/oder um die Enthalpie und/oder um die Wärmekapazität und/oder um die Wärmeleitfähigkeit handelt.

3. Verfahren nach Anspruch 1, wobei

   - für wenigstens eine Basisfunktion ein Rechteckblock gewählt wird.

4. Verfahren nach Anspruch 1, wobei

   - für wenigstens eine Basisfunktion ein Sägezahn gewählt wird.

5. Verfahren nach Anspruch 1, wobei

   - für wenigstens eine Basisfunktion ein B-Spline, vorzugsweise ein B-Spline 4. Ordnung, gewählt wird.

6. Verfahren nach Anspruch 1, wobei

   - für wenigstens eine Basisfunktion eine Gaußfunktion (Gaußglocke) gewählt wird.

7. Verfahren nach Anspruch 1, wobei

   - wenigstens ein Gewichtungsfaktor ($g_i$) die Ausgangsgröße eines neuronalen Netzes ist, dem wenigstens ein Legierungselement und/oder wenigstens eine daraus abgeleitete Größe sowie wenigstens ein Netzgewicht ($w_i$) als Eingangsgrößen zugeführt werden.

8. Verfahren nach Anspruch 1, wobei

- wenigstens ein Gewichtungsfaktor ($g_i$) aus wenigstens einer Linearkombination aus dem Masseanteil wenigstens eines Legierungselementes und/oder daraus abgeleiteter Größen sowie wenigstens einem Regressionsfaktor ($w_i^1$) ermittelt werden.

**9.** Verfahren nach Anspruch 1, wobei

- wenigstens ein Gewichtungsfaktor ($g_i$) aus wenigstens einer Linearkombination aus dem Masseanteil wenigstens eines Legierungselementes und/oder wenigstens eine daraus abgeleitete Größe sowie wenigstens einem freien Parameter ($w_i$) einer nichtlinearen Funktion ($F_i$) gebildet werden.

**Claims**

**1.** Method for determining the thermal materials properties of shaped metal parts from a model which describes the thermal materials properties of the shaped metal part, in which

- at least one thermodynamic parameter (P) is formed as a linear combination of at least one basic function ($h_i$) and at least one weighting factor ($g_i$) in accordance with the relationship

$$p = \pm \sum_{i=1}^{n} g_i \cdot h_i + c, \, n \in N, \, c = \text{const.}$$

or

$$p = \pm \sum_{i=1}^{n} (g_i + c_i) \cdot h_i, \, n \in N, \, c_i = \text{const.}$$

and
- the basic function ($h_i$) describes the thermal materials properties, and
- the weighting factor ($g_i$) takes account of the influence of the alloying elements on at least one thermodynamic parameter (p).

**2.** Method according to Claim 1, in which

- the thermal materials properties which are described by at least one basic function are the temperature and/or the enthalpy and/or the heat capacity and/or the thermal conductivity.

**3.** Method according to Claim 1, in which

- a rectangular block is selected for at least one basic function.

**4.** Method according to Claim 1, in which

- a sawtooth is selected for at least one basic function.

**5.** Method according to Claim 1, in which

- a B spline, preferably a fourth order B spline, is selected for at least one basic function.

**6.** Method according to Claim 1, in which

- a Gaussian function (Gaussian bell) is selected for at least one basic function.

**7.** Method according to Claim 1, in which

- at least one weighting factor ($g_i$) is the output variable of a neural network, to which at least one alloying element and/or at least one variable derived therefrom and at least one network weight ($w_i$) are fed as input variables.

**8.** Method according to Claim 1, in which

- at least one weighting factor ($g_i$) is determined from at least one linear combination of the mass content of at least one alloying element and/or variables derived therefrom and at least one regression factor ($w_i^1$).

**9.** Method according to Claim 1, in which

- at least one weighting factor ($g_i$) is formed from at least one linear combination of the mass content of at least one alloying element and/or at least one variable derived therefrom and at least one free parameter ($w_i$) of a nonlinear function ($F_i$).

**Revendications**

**1.** Procédé de détermination des propriétés thermiques du matériau de pièces métalliques usinées à partir d'un modèle qui décrit les propriétés thermiques du matériau de la pièce métallique usinée, dans lequel

- au moins un paramètre thermodynamique (p) est formé comme combinaison linéaire d'au moins une fonction de base ($h_i$) et d'au moins un facteur de pondération ($g_i$) selon la relation

$$p = \pm \sum_{i=1}^{n} g_i \cdot h_i + c, \quad n \in N, \ c = const.$$

ou

$$p = \pm \sum_{i=1}^{n} (g_i + c_i) \cdot h_i, \quad n \in N, \ c_i = const.$$

- la fonction de base ($h_i$) décrit les propriétés thermiques du matériau, et
- le facteur de pondération ($g_i$) prend en compte l'influence des éléments d'alliage sur au moins un paramètre thermodynamique (p).

**2.** Procédé selon la revendication 1, dans lequel

- les propriétés thermiques du matériau qui sont décrites par au moins une fonction de base sont la température et/ou l'enthalpie et/ou la capacité thermique et/ou la conductibilité thermique.

**3.** Procédé selon la revendication 1, dans lequel

- une forme de bloc rectangulaire est choisie pour au moins une fonction de base.

**4.** Procédé selon la revendication 1, dans lequel

- une forme de dents de scie est choisie pour au moins une fonction de base.

**5.** Procédé selon la revendication 1, dans lequel

**EP 1 314 028 B1**

- une forme de B-spline, de préférence de B-spline d'ordre 4, est choisie pour au moins une fonction de base.

6. Procédé selon la revendication 1, dans lequel

- une forme de fonction de Gauss (cloche de Gauss) est choisie pour au moins une fonction de base.

7. Procédé selon la revendication 1, dans lequel

- au moins un facteur de pondération ($g_i$) est la grandeur de sortie d'un réseau neuronal auquel sont envoyés comme grandeurs d'entrée au moins un élément d'alliage et/ou au moins une grandeur déduite de celui-ci ainsi qu'au moins un poids de réseau ($w_i$).

8. Procédé selon la revendication 1, dans lequel

- au moins un facteur de pondération ($g_i$) est déterminé à partir d'au moins une combinaison linéaire du rapport massique d'au moins un élément d'alliage et/ou de grandeurs déduites de là ainsi que d'au moins un facteur de régression ( $w_i^1$ ) .

9. Procédé selon la revendication 1, dans lequel

- au moins un facteur de pondération ($g_i$) est formé à partir d'au moins une combinaison linéaire du rapport massique d'au moins un élément d'alliage et/ou d'au moins une grandeur déduite de là ainsi que d'au moins un paramètre libre ($w_i$) d'une fonction non linéaire ($F_i$).

9

FIG 1

EP 1 314 028 B1

$w_1^i$   $w_2^i$   $w_3^i$   $w_4^i$   $w_5^i$   $w_6^i$

**FIG 2**

$w_j^i$

$F_i$

$g_i$

**FIG 3**